(19) Europäisches Patentamt
European Patent Office
Office européen des brevets

(11) Numéro de publication : 0 524 961 B1

(12) FASCICULE DE BREVET EUROPEEN

(45) Date de publication du fascicule du brevet : **06.07.94 Bulletin 94/27**

(51) Int. Cl.$^5$ : **A61K 31/655,** C07C 281/20

(21) Numéro de dépôt : **91906301.6**

(22) Date de dépôt : **08.03.91**

(86) Numéro de dépôt international : **PCT/EP91/00445**

(87) Numéro de publication internationale : **WO 91/16054 31.10.91 Gazette 91/25**

# (54) DERIVES AZOIQUES, COMPOSITIONS PHARMACEUTIQUES ET DESINFECTANTES LES CONTENANT ET LEURS UTILISATIONS CONTRE LE SIDA.

(30) Priorité : **19.04.90 BE 9000435**

(43) Date de publication de la demande : **03.02.93 Bulletin 93/05**

(45) Mention de la délivrance du brevet : **06.07.94 Bulletin 94/27**

(84) Etats contractants désignés : **AT BE CH DE DK ES FR GB GR IT LI LU NL SE**

(56) Documents cités :
**FR-A- 2 056 874**
**US-A- 3 225 026**
**US-A- 3 684 713**
**JOURNAL OF THE AMERICAN CHEMICAL SOCIETY, Vol. 56, 1934, Washington, US; F.C.SCHMELKES et al.: "N,N'-Dichloroazoazodicarbonamidine (Azochloramid), an N-chloro derivative of the oxidant in an oxidation-reduction system", seethewhole document cited in the application.**
**JOURNAL OF THE AMERICAN CHEMICAL SOCIETY, Vol. 75, No. 13, 1953, Washington,US; W.D. KUMLER: "The dipole moments, ultraviolet spectra and structure of azo-bis-(chloroformamidine) and azo-bis-(nitroformamidine)", pages 3092-3093, seethe whole document cited in the application.**
**PROC. NATL. ACAD. SCI. USA, Vol. 78, No. 6, June 1981, Biochemistry,Washington, US; H. SIES et al.: "Hepatic calcium efflux during cytochrome p-450-dependent drug oxidations at the endoplasmic reticulum in intact liver",pages 3358-3362, see page 3361, figure 5.**

(56) Documents cités :
**MARTINDALE THE EXTRA PHARMACOPOEIA, Edition 28, 1982, The Pharmaceutical Press,London, GB, page 558, No. 2225-n: Chloroazodin**
**J.GEN.VIROL., Vol. 68, No. 8, 1987, GB; K.D. JENTSCH et al.: " Inhibition ofhuman immunodifiency virus type I reverse transcriptase by suraminrelatedcompounds", pages 2183-2192, see whole document, in particular table 2**

(73) Titulaire : **PREVISAN S.A.**
**14, bd. Emmanuel Servais**
**Luxembourg (LU)**

(72) Inventeur : **VANDEVELDE, Michel**
**14, avenue des Bouvreuils**
**B-1301 Bierges (BE)**
Inventeur : **MARGERY, Hélène**
**14, avenue des Bouvreuils**
**B-1301 Bierges (BE)**

(74) Mandataire : **Claeys, Pierre et al**
**GEVERS Patents,**
**Rue de Livourne 7,**
**Boîte Postale 1**
**B-1050 Bruxelles (BE)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

La présente invention est relative à des dérivés azoïques et aux compositions pharmaceutiques et de désinfection contenant ces dérivés.

Les rétrovirus sont définis suivant l'invention comme des virus dont le matériel génétique porté par une chaîne d'acide ribonucléique est transcrit à l'intérieur d'une cellule cible en acide désoxyribonucléique grâce à une enzyme appelée transcriptase inverse.

Ces virus sont responsables de pathologies dans le monde végétal et animal. Une liste non exhaustive de ces virus est donnée dans J.M. HURAUX et cons., Virologie. Flammarion Médecine-Sciences, 1985, Paris.

Lorsque le stade d'intégration au sein des chromosomes de la cellule cible est acquis, les chances de guérison (retour à l'état antérieur) sont faibles. En effet, ces virus infectent des lignées cellulaires de différents types et aucun médicament susceptible d'extraire le matériel génétique viral des cellules infectées ne semble probable à l'heure actuelle.

Par ailleurs, ces virus ont des propriétés de mutation et ils sont abrités par des réservoirs animaux qui peuvent leur permettre de se présenter sous des formes antigéniques nouvelles (par utilisation de fragments cellulaires de la cellule hôte, par exemple), ce qui rend la vaccination complexe.

A ce jour, un seul traitement est connu qui prolonge la survie des patients, sans permettre toutefois la guérison. Ce traitement comprend l'administration de 3′-azido-3′-désoxythymidine (voir EP-A-196185). Cette substance agit par inhibition de la transcriptase inverse.

D'autres substances sont connues pour inhiber la réplication des virus VIH par leur action sur la transcriptase inverse. On peut par exemple citer certains didésoxynucléotides (voir EP-A-307914).

On a aussi découvert que certaines substances avaient pour effet de bloquer la pénétration du virus dans les cellules. Comme substances ayant cet effet, on peut citer par exemple des oligosaccharides ou polysaccharides (voir EP-A-240098) ou encore de la castonospermine (B.D. WALKER, Inhibition of human Immunodeficiency virus syncytium formation and virus replication by castonospermine, Proc. Natl. Acad. Sci. USA, vol.84, p. 8120-8124, Nov. 1987).

Selon tous ces traitements, on peut espérer que le virus ayant infecté un patient ne pourra poursuivre son développement et sa propagation. On ne revient toutefois pas à l'état antérieur à l'affection, car le provirus n'est pas atteint et il subsiste dans la cellule auparavant touchée. Le traitement est donc palliatif, et non curatif.

Ce type de traitement présente le grand danger de permettre l'apparition de résistances des virus aux composés. Il semble déjà établi que le virus devient résistant à la 3′-azido-3′-désoxythymidine à plus ou moins long terme, notamment après environ 12 à 18 mois (SCHULHAFER E. et cons., Acquired immunodeficiency syndrome...... In Vivo 3(2): 61-78(1989)).

On a enfin déjà envisagé, comme médicament pour combattre les rétrovirus, des dérivés de la benzidine, qui portent entre autres des groupes azoïques (voir FR-A-2612515). Il ne ressort toutefois de ce document qu'une simple affirmation à propos de l'activité de ces composés.

Les modes de transmission des virus ont été établis lorsqu'il y avait eu contact sanguin direct et contact au travers de plaies par du matériel infecté. Le risque de transmission par des objets et notamment du matériel médical infecté est non négligeable.

Par ailleurs, le mode de transmission sexuel et la transmission aux enfants par le lait maternel infecté sont aussi établis ce qui montre le passage possible de particules infectantes à travers des muqueuses saines (P. LEPAGE et cons.. Postnatal Transmission of HIV from Mother to Child, The Lancet, August 15, 1987, p. 400; C.J. MILLER et cons., Genital Mucosal Transmission of Simian Immunodificiency Virus, Journal of Virology, Oct. 1989, p. 4277-4284).

Il semble de plus en plus probable qu'il puisse exister une inoculation du virus par simple contact, c'est-à-dire à travers la peau ou une muqueuse. Certains chercheurs ont émis l'opinion que les virus inoculés sembleraient alors connaître une phase de réplication pendant laquelle ils restent dans la sphère de la muqueuse ou de la peau du porteur. Cette phase pourrait se prolonger pendant plusieurs mois. Ce ne serait que dans une seconde phase que les virus et/ou ses constituants dissémineraient à partir de la muqueuse (R. ZITTOUN, Syndrome Immuno Déficitaire Acquis, Doins Editeurs, Paris, 1986, p. 183-184).

Ainsi la mise au point de molécules permettant la désinfection de surfaces et d'objets inanimés ainsi que de matières et produits venant au contact des muqueuses et de la peau s'est avérée nécessaire pour l'éradication de la maladie. Il est apparu en effet indispensable d'empêcher dans la mesure du possible la transmission des rétrovirus entre un porteur et une personne saine.

On connaît déjà dans ce but des compositions et un procédé de désinfection à base d'oligosaccharides ou de polysaccharides naturels ou synthétiques ayant au moins un groupe S-oxoacide (voir EP-A-285357). Il résulte toutefois clairement des exemples donnés que, même si ces compositions sont actives contre les rétrovirus, elles laissent toujours subsister une partie des virus présents, ce qui, à terme, présente l'énorme

danger de voir l'apparition d'une population de virus résistants, encore plus dangereux.

Dans une demande de brevet internationale WO-A-90/01935 on a déjà prévu des produits susceptibles d'entrer localement en contact avec la peau, les muqueuses ou des sécrétions corporelles, ces produits comprenant un agent actif pour combattre les virus du groupe rétrovirus, par exemple de la suramine sodique, ainsi que quelques dérivés azoïques complexes comme le pyridium, la néotropine, le rouge Congo, le bleu de trypan, le rouge de trypan et le violet de trypan.

On a par ailleurs examiné l'action vis-à-vis des rétrovirus des désinfectants chimiques courants, tels que l'éthanol, le glutaraldéhyde, l'hypochlorite de sodium, la formaline, la β-propio- lactone, l'alcool dénaturé, entre autres (V.B. SPIRE et cons., Inactivation of Lymphadenopathy associated virus by chemical disinfectants. The Lancet, October 20, 1984, p. 899-901; L. RESNICK et cons., Stability and inactivation of HTLV-III/LAV under clinical and laboratory environments, JAMA, 11 avril 1986, Volume 255, n° 14; L.S. MARTIN et cons., Disinfection and inactivation of the human T lymphotropic Virus type III/lymphadenopathy-associated virus, The Journal of Infectious Diseases, Vol. 152, n° 2, aout 1985; P.J.V. HANSON et cons., Chemical inactivation of HIV on surfaces, Br. Med. J., 1989, 298 : 862-4).

Il ressort de ces examens que la plupart des désinfectants utilisés en milieu hospitalier sont inefficaces ou peu efficaces vis-à-vis des rétrovirus VIH, et donc potentiellement dangereux. Ceux qui se sont montrés les plus efficaces demandent des temps de contact assez longs, parfois 10 minutes et davantage, ce qui est matériellement difficile en cas de nettoyage de sols, de tables, par exemple. Enfin, certains de ces désinfectants semblent perdre leur efficacité en présence de milieux protéiques ou sont inapplicables s'ils doivent venir au contact de la peau ou des muqueuses d'un corps vivant, étant donné leur agressivité chimique ou leur toxicité cellulaire.

La présente invention a par conséquent pour but de mettre au point un agent actif pour combattre les virus, notamment du groupe rétrovirus, en particulier les virus d'immunodéficience humaine VIH, qui soit capable d'agir radicalement sur ces virus, de préférence avec une élimination complète de ceux-ci, et en maintenant son activité à de très faibles concentrations. Avantageusement, cet agent conservera son potentiel d'action en milieu cellulaire, en milieu aqueux, ainsi qu'en présence de milieux organiques, en particulier protéiques. Sa toxicité sera de préférence peu élevée. Sous une forme préférentielle, l'action virucide sera très rapide envers les virus VIH.

L'invention a également pour but de mettre au point une composition pharmaceutique permettant le traitement ou la prophylaxie des maladies virales.

L'invention a aussi pour but la préparation d'une composition de désinfection d'objets inanimés à l'encontre des virus.

Il est évident que, selon ses applications, que ce soit comme substance active dans une composition pharmaceutique, ou comme agent désinfectant dans un produit de nettoyage, une composition cosmétique ou d'autres produits de ce genre, l'agent actif devra répondre à des exigences différentes de solubilité, de toxicité ou de stabilité. Il en sera de même si l'application de la composition pharmaceutique doit se faire par voie orale, parentérale, intraveineuse, locale ou autre, ou si la désinfection concerne des objets inanimés du type instruments ou sols ou au contraire des déchets organiques ou des liquides à absorber.

On a à présent découvert que, d'une manière surprenante, certains composés azoïques sont capables de jouer de manière particulièrement efficace et radicale le rôle de l'agent actif recherché.

Pour résoudre les problèmes posés, il est prévu suivant l'invention des dérivés azoïques répondant à la formule générale

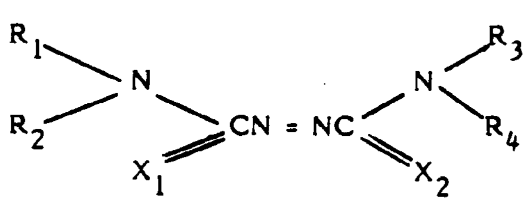

dans laquelle $R_1$ à $R_4$ sont identiques ou différents et représentent chacun un atome d'hydrogène ou d'halogène ou un radical d'hydrocarbure aliphatique ou aromatique, éventuellement substitué, comportant de 1 à 6 atomes de carbone, $X_1$ et $X_2$ sont identiques ou différents et représentent chacun un atome d'oxygène ou un groupe $NR_5$, où $R_5$ représente un atome d'hydrogène ou d'halogène, un radical d'hydrocarbure aliphatique ou aromatique comportant de 1 à 6 atomes de carbone ou un groupe nitro, $R_5$ pouvant, en présence de deux groupes $NR_5$ simultanément, présenter une signification identique ou différente dans chacun de ces groupes et $R_5$ ayant une signification autre que celle d'atome de chlore simultanément dans les deux groupes $NR_5$ lorsque $R_1$ à $R_4$ représentent de l'hydrogène, ainsi que leurs isomères, le 1,1'-azobisdiméthylformamide étant exclu de ces dérivés, pour leur application en tant que substances thérapeutiquement actives.

Suivant l'invention, dans ces dérivés, $R_1$ à $R_5$ peuvent avantageusement représenter, indépendamment en soi, un radical d'hydrocarbure aliphatique inférieur, en particulier un groupe méthyle, éthyle, propyle ou butyle. On peut aussi prévoir des groupes benzyle. Dans les composés suivant la formule générale, les atomes d'halogène sont en particulier ceux de chlore, de brome, d'iode ou de fluor.

Comme dérivés azoïques suivant l'invention on envisage en particulier le 1,1'-azobisformamide, la 1,1'-azobisformamidine, la 1,1'-azobisnitroformamidine.

Comme on le verra dans la suite, le 1,1'-azobisdiméthylformamide n'est pas exclu de l'invention lorsque celle-ci concerne l'utilisation des dérivés répondant à la formule générale.

Il faut entendre que l'invention n'est pas limitée aux dérivés 1,1' et que ceux en position 2,2' sont également compris dans celle-ci, ainsi que tous les isomères et leurs mélanges.

La préparation de la 1,1'-azobisformamidine et du 1,1'-azobisformamide a déjà été réalisée à la fin du siècle passé par J. THIELE (v. The Merck Index, 10 éd., 919, Rahway, 1983; F.C. SCHMELKES et cons., N, N'-Dichloroazodicarbonamidine (azochloramid), a N-chloro derivative of the oxidant in an oxidation-reduction system, Journal of American Chemical Society, 56, 1610, 1934; FR-B-2056874; US-A-3225026; US-A-3684713). Le 1,1'-azobisformamide est connu comme adjuvant dans la farine alimentaire. Le 1,1'-azobisdiméthylformamide est également connu depuis longtemps par son action oxydante intracellulaire sur le glutathion de cellules du sang humain (N.S. KOSOWER et cons., Diamide, a new reagent for the intracellular oxidation of glutathione to the disulfide, Biochemical and Biophysical Research Communications, vol. 37, n° 4, 1969) ainsi que par son initiation d'un efflux de $Ca^{2+}$ supplémentaire depuis le foie de rats perfusé (H. SIES et cons., Hepatic calcium efflux during cytochrome P-450-dependent drug oxidations at the endoplasmic reticulum in intact liver, Proc. Natl. Acad. Sci. USA, Vol. 78, n° 6, p. 3358-3362). On connaît également depuis longtemps la 1,1'-azobisnitroformamidine (W.D. KUMLER, The Dipole Moments, Ultraviolet spectra and structure of azo-bis-(chloroformamidine) and azo-bis-(nitroformamidine), Journal of American Chemical Society, 75, 3092, 1953). Il ressort clairement de cet état connu de la technique qu'il était tout à fait inattendu d'obtenir l'effet recherché à partir de ces substances relativement simples, peu coûteuses à fabriquer et connues depuis très longtemps.

Un effet encore plus inattendu a pu être observé. Il est apparu que ces dérivés montrent une toxicité sélective vis-à-vis des cellules infectées par des virus VIH, alors que les cellules Supt-1 et lymphocytaires du corps humain ne sont pas ou peu altérées par les composés testés. Ces observations permettent d'envisager la possibilité d'une chimiothérapie détruisant sélectivement les cellules infectées en épargnant les cellules saines du patient. On peut donc envisager un retour à l'état antérieur du patient infecté, c'est-à-dire une guérison de ce dernier.

On prévoit donc suivant l'invention une composition pharmaceutique renfermant, comme substance active, au moins un dérivé azoïque répondant à la formule générale de la revendication 1 ou un isomère pharmaceutiquement acceptable de ces dérivés, et au moins un excipient pharmaceutiquement compatible, ainsi qu'éventuellement un ou plusieurs adjuvants courants en pharmacie. Cette composition peut être administrée sous quelque forme que ce soit, par voie orale ou sublinguale, rectale ou vaginale, par injection ou perfusion, par voie locale, transcutanée ou transmuqueuse, ou par toute autre forme courante en médecine thérapeut ique ou vétérinaire. L'excipient et des adjuvants courants éventuels sont sélectionnés en fonction du mode d'administration choisi. Avantageusement des agents de conservation ou de solubilisation, de neutralité du pH, d'isotonicité, de tamponnage ou autres peuvent être ajoutés à la composition.

L'excipient ou support choisi peut être solide ou liquide. La composition se présentera au choix sous forme de poudre, d'onguent, de comprimés, de gélules, de capsules, d'aérosols, de liquide à injecter, etc....

On peut aussi prévoir des formules qui libèrent la substance active avec retard.

Suivant une forme de réalisation avantageuse de l'invention, la composition pharmaceutique suivant l'invention comprend en supplément un désinfectant. En effet, en plus de son effet curatif, il peut être avantageux pour le porteur de virus que la composition le défende contre des agressions extérieures qui, en stimulant son système de défense immunitaire, favorisent le prolifération des virus VIH qu'il porte.

On a également prévu suivant l'invention l'utilisation de ces mêmes dérivés azoïques, répondant à la formule précitée, y compris le 1,1'-azobisdiméthylformamide, pour combattre sur et/ou dans des objets inanimés les virus, notamment du groupe rétrovirus, en particulier les virus d'immunodéficience humaine VIH.

Il est prévu aussi, lors de cette utilisation suivant l'invention, la mise en oeuvre de compositions de désinfection d'objets inanimés contenant au moins un de ces dérivés azoïques actifs ainsi qu'un support approprié. Comme support, on peut avantageusement prévoir de l'eau ou un quelconque autre solvant approprié comme véhicule dans lequel l'agent actif est en solution. D'autres agents désinfectants ou adjuvants courants peuvent être prévus éventuellement en supplément.

Suivant l'invention, on prévoit l'utilisation des dérivés azoïques actifs ou des compositions de désinfection

précitées pour la désinfection, vis-à-vis des virus, notamment du groupe rétrovirus, en particulier des virus d'immunodéficience humaine VIH, d'objets inanimés.

Comme objets inanimés à désinfecter, on peut prévoir, dans l'utilisation suivant l'invention, par exemple:

- des matériels sanitaires en matière plastique, textile ou caoutchouteuse : ouate, coton absorbant, gaze, bandages, papier hygiénique, films d'emballage, etc.
- des instruments et appareils médicaux, vétérinaires et de dentisterie : seringues, canules, sondes, pinces, ciseaux, trousses de lavage d'estomac, tables de chirurgie, bassins, etc...
- des vêtements médicaux, vétérinaires ou de dentisterie : gants, chemises, serviettes, etc....
- des objets nécessitant une manipulation dans le domaine de l'alimentation : biberons, casseroles, bouteilles ou boîtes, notamment pour des boissons, etc....
- des instruments de cosmétologie : matériel et outillage de coiffure, de manucure, de pédicure, d'esthéticien, etc....
- des véhicules sanitaires : ambulances, tables roulantes, etc....
- des surfaces de sol et de paroi : locaux, en particulier en milieu hospitalier, etc....
- des appareils sanitaires et hygiéniques : lavabos, pannes, cuvettes, baignoires, etc...
- des boissons : eaux nécessaires à des boissons, lait, etc...
- des eaux de piscine.

On peut aussi prévoir la désinfection des excréments, des résidus d'analyses de laboratoire et notamment d'échantillons prélevés du corps humain ou animal, par exemple en vue d'une analyse.

Il est avantageux de prévoir, suivant l'invention, que certaines compositions cosmétiques puissent également contenir au moins un dérivé azoïque actif suivant l'invention. Dans ce cas évidemment, divers support et adjuvants courants dans ce domaine peuvent être mis en application.

Il est prévu, suivant l'invention, une utilisation particulière d'un agent actif ou d'une composition active suivant l'invention dans ou sur des produits susceptibles d'entrer en contact avec la peau ou les muqueuses d'un corps humain ou animal, éventuellement porteur de virus. Dans ou sur certains de ces produits, comme des gants de chirurgien ou de dentiste, des pessaires, des condoms, etc....., en plus de son action de désinfection, l'agent ou la composition active peut avantageusement former un milieu de barrière à la transmission des rétrovirus depuis le porteur vers une personne saine. On peut par exemple prévoir de lubrifier un condom en matière caoutchouteuse à l'aide d'une vaseline renfermant un agent actif suivant l'invention. On peut aussi, pour des gants de chirurgien, prévoir deux pellicules caoutchouteuses entre lesquelles une poudre d'amylase amylopectine par exemple contenant un agent actif suivant l'invention est enfermée. Dans ce dernier cas, la poudre désinfectante, servant de barrière, n'est donc pas elle-même en contact direct avec la peau.

La composition de désinfection suivant l'invention peut aussi contenir éventuellement en supplément un agent désinfectant, de préférence à large spectre d'action germicide. La composition obtenue présente ainsi une défense appréciable contre la présence d'agents pathogènes ou allogènes, autres que les rétrovirus VIH.

Cette dernière propriété est très importante non seulement par la large action de désinfection obtenue, mais aussi parce qu'elle peut être utile au porteur du virus lui-même. En effet, celui-ci doit éviter autant que possible toute activation de ses cellules lymphocytaires. Cette activation, chez le porteur de VIH, a pour effet la réplication du virus et la prolifération de ce dernier dans ses cellules. Un porteur de VIH a donc tout avantage à suivre des habitudes de vie hygiéniques qui écartent au maximum tout danger d'activation des cellules infectées, et par conséquent une réaction immunitaire de son organisme.

L'utilisation des agents et compositions suivant l'invention lui offre la possibilité de se désinfecter, mais aussi en supplément celle de le protéger contre des réactions immunitaires d'une autre origine.

Il est aussi prévu suivant l'invention, d'utiliser les dérivés azoïques répondant à la formule générale, y compris le 1,1'-azobisdiméthylformamide, pour la fabrication de médicaments à mettre en oeuvre dans le traitement ou la prophylaxie des maladies virales, notamment pour les infections par les rétrovirus.

L'invention va à présent être décrite de manière plus détaillée à l'aide de quelques exemples de réalisation non limitatifs.

**Exemple 1**

Comprimés

| | |
|---|---|
| 1,1'-azobisdiméthylformamide | 200 à 300 mg |
| cellulose microcristalline (avicel PH 101) | 60 mg |
| povidone BP | 15 mg |
| glycollate d'amidon sodique | 20 mg |
| stéarate de magnésium | 5 mg |
| lactose | q.s. pour 500 mg. |

**Exemple 2**

Capsules

| | |
|---|---|
| 1,1'-azobisformamide | 400 à 600 mg |
| cellulose microcristalline (avicel) | 50 mg |

pour une capsule.

**Exemple 3**

Formule à délitage retard sur 6 à 8 heures.

| | |
|---|---|
| 1,1′-azobisdiméthylformamide | 400 à 600 mg |
| hydroxypropylméthylcellulose | 111 mg |
| lactose | 53 mg |
| povidone BP | 28 mg |
| stéarate de magnésium | 7 mg. |

On mélange l'hydroxypropylméthylcellulose au 1,1′-azobisdiméthylformamide en premier lieu, puis on ajoute les autres composants.

**Exemple 4**

Forme injectable.

| | |
|---|---|
| 1,1′-azobisformamide | 10 à 30 mg |
| acide chlorhydrique | 0,1 M |
| hydroxyde de sodium | 0,1 M |

On ajuste le pH à une valeur de 4 à 7 à l'aide soit de l'acide, soit de la base, on dissout le produit à chaud et on filtre sur micropore stérile. Le liquide est alors transvasé dans des ampoules stériles brunes.

**Exemple 5**

Forme injectable.

| | |
|---|---|
| 1,1′-azobisformamidine | 50 à 150 mg |
| alcool benzylique | 10 mg |
| glycofurol | 75 mg |
| eau pour injection | 3ml |

Le produit est réalisé par dissolution de la substance active dans le glycofurol, puis par mélange de la solution obtenue à de l'eau filtrée et à de l'alcool benzylique. On transvase le tout dans une ampoule brune stérile.

**Exemple 6**

Forme pour injection par voie intraveineuse.

| | |
|---|---|
| 1,1′-azobisnitroformamidine | 50 à 150 mg |

On ajoute à ce composant de l'eau stérile sans pyrogène, tamponnée par un tampon phosphate à pH 7, pour obtenir 25 ml.

**Exemple 7**

Suppositoire
A 150 à 250 g de poudre micronisée de 1,1'-azobisformamidine, on ajoute 2g de glycérine pour former un suppositoire.

Des essais expérimentaux ont été effectués par le laboratoire de l'Institut Pasteur du Brabant pour examiner l'efficacité des dérivés azoïques suivant l'invention.

**Exemple 8**

**Matériel de départ de l'essai:**

- de la 1,1'-azobisformamidine d'un degré de pureté de 98%. La pureté a été examinée par un test de carbone-hydrogène.
- un surnageant de cultures de cellules (Molt-3) continuellement productrices de virus VIH-1. Le surnageant viral utilisé a une activité de transcriptase inverse de 1 x $10^6$ cpm/ml.
- des cellules d'une lignée T humaine continue de phénotype $T_4$ (Supt-1) cultivée en milieu RPMI, additionné de 10 % de sérum de veau foetal et de 1 % de glutamine.

L'efficacité de la 1,1'-azobisformamidine a été étudiée à deux niveaux :

- sa toxicité vis-à-vis des cellules saines.
- son action sur l'infectivité du virus VIH-1 pour la lignée Supt-1.

**a)** Examen de la toxicité vis-à-vis de cellules saines.

On détermine la mortalité cellulaire par exclusion au bleu de trypan. A des dilutions de 1/500, 1/1500 et 1/3000, la 1,1'-azobisformamidine ne diminue pas la viabilité cellulaire de cellules Supt-1.

**b)** Examen de l'infectivité.

On fait incuber des préparations virales de VIH-1 à haut titre en présence de dilutions de 1,1'-azobisformamidine de 1/500, de 1/1500 et de 1/3000 et pendant différents temps d'incubation : 1 minute, 10 minutes et 30 minutes. Pour l'incubation on met en présence 0,3 ml de concentré de virus et la substance active à une concentration doublée (une fois pour le volume de virus et une fois pour le volume de produit). Pendant 30 minutes à 37°C, on traite préalablement les cellules Supt-1 par 10 µg/ml de polybrène, puis on les inocule avec les préparations de virus traités. On prévoit comme témoins, des cellules non inoculées, et des cellules inoculées par une préparation de virus non traités.

On détermine ensuite l'infectivité, à chaque passage cellulaire, de ces préparations en suivant la production virale (mesure de l'antigène p24 exprimé) dans les lysats cellulaires solubilisés des cellules Supt-1 examinées.

Les mesures du pouvoir infectieux du virus VIH-1 après incubation avec de la 1,1'-azobisformamidine ressortent du tableau I ci-dessous, en comparaison d'un témoin non infecté et d'un témoin infecté par des virus non traités.

**Exemple 9**

Le matériel de départ de l'essai se distingue de celui de l'exemple 8 par l'utilisation de 1,1'-azobisdiméthylformamide, au lieu de 1,1'-azobisformamidine.

**a)** Examen de la toxicité.

A des dilutions de 1/500, 1/1500 et 1/3000, le 1,1'-azobisdiméthyl- formamide ne diminue pas la viabilité cellulaire des cellules Supt-1.

**b)** Examen de l'infectivité.

On procède de la même manière que dans l'exemple 8b.

Les mesures du pouvoir infectieux du virus VIH-1 après incubation avec du 1,1'-azobisdiméthylformamide ressortent du tableau 1 suivant.

**Tableau 1**

Mesure d'infectivité du virus VIH 1 au 14ème jour après l'inoculation de cellules Supt-1.

| Substance active (dilution) | Durée de traitement des virus | |
|---|---|---|
| 1,1'-Azobisformamidine | | |
| 1/500 | 1 min | 0,234 ± 0,008 |
| | 10 min | 0,267 ± 0,021 |
| | 30 min | 0,245 ± 0,020 |
| 1/1500 | 1 min | 0,469 ± 0,013 |
| | 10 min | 0,439 ± 0,008 |
| | 30 min | 0,406 ± 0,032 |
| 1/3000 | 1 min | 0,809 ± 0,049 |
| | 10 min | 0,505 ± 0,023 |
| | 30 min | 0,740 ± 0,013 |
| 1,1'-Azobisdiméthylformamide | | |
| 1/500 | 1 min | 0,275 ± 0,049 |
| | 10 min | 0,206 ± 0,006 |
| | 30 min | 0,203 ± 0,008 |
| 1/1500 | 1 min | 0,278 ± 0,050 |
| | 10 min | 0,217 ± 0,028 |
| | 30 min | 0,193 ± 0,004 |
| 1/3000 | 1 min | 0,239 ± 0,016 |
| | 10 min | 0,283 ± 0,042 |
| | 30 min | 0,198 ± 0,009 |
| Témoin virus | | 1,033 ± 0,081 |
| Témoin cellule | | 0,206 ± 0,005 |

(densité optique 492 nm).

Il ressort clairement de ce tableau qu'à une dilution de 1/500 la 1,1′-azobisformamidine protège les cellules Supt-1 après un traitement des virus de seulement 1 minute. Le 1,1′-azobisdiméthylformamide a cet effet même à des dilutions de 1/3000. Pour cette dernière substance le test a été prolongé jusqu'au 25ème jour. L'efficacité de cette substance active est maintenue pour toutes les dilutions, lorsque les virus ont été traités pendant 30 minutes.

Au microscope en contraste de phase, on n'a par ailleurs observé aucun effet cytopathogène pour les cellules inoculées avec du virus prétraité au 1,1′-azobisdiméthylformamide et à la 1,1′-azobisformamidine. Par contre des syncytia apparaissent dès le 14ème jour après l'infection avec la préparation virale témoin.

**c)** Le 1,1'-azobisdiméthylformamide a en outre été examiné à propos de sa toxicité envers les cellules infectées par des virus.

Dans ce but, on détermine la mortalité cellulaire par exclusion au bleu trypan.

Cet examen est effectué sur des cellules Molt-3 infectées par HTLVIII-B, qui sont productrices de virus VIH-1.

**Tableau 2**

| Cellules Molt-3 (VIH 1) | | Nombre de cellules vivantes | Nombre de cellules mortes | % des cellules mortes |
|---|---|---|---|---|
| Témoins | | 57 | 5 | 8 |
| Cellules traitées par du 1,1'-azobisdiméthylformamide | | | | |
| Dilution | Durée (min.) | | | |
| 1/500 | 1 | 62 | 9 | 12,7 |
| | 10 | 67 | 11 | 14 |
| | 30 | 58 | 12 | 17,1 |
| 1/1500 | 30 | 70 | 15 | 17.6 |
| 1/3000 | 30 | 58 | 12 | 17,1 |

Il ressort très clairement du tableau 2 que la mortalité des cellules infectées est doublée en présence de 1.1'-azobis- diméthylformamide après 30 minutes d'action, même à la dilution de 1/3000, alors que cette molécule ne montre aucune toxicité pour les lignées cellulaires Supt-1 et lymphocytaires humaines, même après 24 heures.

## Exemple 10

Le matériel de l'essai se distingue de ceux de l'exemple 9 en ce que le 1,1'-azobis-diméthylformamide est dosé à une dilution de 1/100 (10 mg/ml).

**a)** Examen de la toxicité cellulaire vis-à-vis de cellules normales, c'est-à-dire non infectées (lignée Supt-1).

On détermine la mortalité cellulaire par exclusion au bleu de trypan.

Tableau 3

| Cellules Supt-1 | Durée (heures) | Nombre de cellules vivantes | Nombre de cellules mortes | % de cellules mortes |
|---|---|---|---|---|
| Temoins | 0 | 61 | 19 | 24 |
| | 1 | 57 | 18 | 32 |
| | 5 | 55 | 20 | 36 |
| Cellules traitées par du 1,1'-azobisdiméthylformamide (dilution: 1/100) | 0 | 61 | 21 | 26 |
| | 1 | 63 | 20 | 24 |
| | 5 | 56 | 22 | 28 |

Ces résultats sont donnés par millilitre de culture prélevée.

Il ressort clairement du tableau que, même à une concentration relativement haute, le 1,1′-azobisdiméthylformamide ne présente pas de toxicité vis-à-vis des cellules Supt-1 saines.

**b)** Examen de l'infectivité du virus VIH-1.

On procède comme dans l'exemple 8 b), en traitant les virus pendant 30 minutes avec le 1,1′-azobisdiméthylformamide à une dilution de 1/100 Après inoculation de cellules Supt-1 par cette préparation virale, on détermine les antigènes p24 exprimés par densité optique, après 14, 18 et 21 jours.

Tableau 4

Antigènes p24 exprimés par densité optique.

| | Jour 14 | Jour 18 | Jour 21 |
|---|---|---|---|
| Cellules témoins | 0,125 | 0,126 | 0,168 |
| Cellules témoins infectées par VIH | 0,224 | 1,078 | 0,765 |
| Cellules - VIH-1 traité par la substance active | 0,144 | 0,160 | 0,130 |

Il ressort de cette expérience que le VIH traite par le 1,1′-azobisdiméthylformamide est incapable d'infecter les cellules saines Supt-1.

**c)** Examen de l'intégration du génome viral dans l'ADN chromosomique de cellules Supt-1.

Par une amplification génétique grâce à une polymérase thermostable on met en évidence la présence des gènes "GAG" "LTR" et "ENV" du provirus VIH-1. Cette méthode est appelée P.C.R. (Polymérase Chain Reaction) (Chin ·Yih Ou et cons., Sciences 239, 295-297, 1988).

Cette méthode est appliquée pour détecter ces provirus VIH-1 dans le génome de cellules Supt-1 inoculées par des virus VIH-1 mis à incuber avec du 1,1′-azobisdiméthylformamide, (dilution 1/100), comme décrit dans l'exemple 8.

Des oligonucléotides amplifiés, visibles aux UV au moyen de bromure d'éthidium, apparaissent seulement dans les tubes correspondant à une inoculation avec des virus VIH qui n'ont pas été traités par la substance active.

De plus, après hybridation avec des sondes marquées au $P^{32}$ spécifiques pour les trois gènes recherchés, il est possible de conclure que le provirus VIH-1 est totalement absent à l'intérieur des cellules Supt-1 qui ont reçu des inoculats de virions VIH-1 traités par une dilution de 1/100 de la substance active.

On peut donc déduire que cette substance active protège les cellules contre l'infection par le VIH-1.

**Exemple 11**

Le matériel de l'essai se distingue de celui de l'exemple 8 en ce que la substance utilisée est du 1,1′-azobisformamide dosé à une concentration de 35 mg/litre, soit 35 µ/ml, c'est-à-dire à une concentration extrêmement faible (obtenue par le surnageant d'une suspension à 0,5 g/litre dans du RPMI).

**a)** Examen de la toxicité cellulaire vis-à-vis des cellules saines.

Aucun effet toxique n'a été noté pour les cellules Supt-1, même après 24 heures d'incubation.

**b)** Examen de l'infectivité.

On procède comme dans l'exemple 8 b) avec des temps d'incubation des virus de 15 minutes, 30 minutes, 60 minutes et 120 minutes. Aucun effet cytopathogène n'a été observé (pas de formation de syncytia).

Tableau 5

Antigènes p 24 exprimés (densité optique).

| | Jour 14 | Jour 18 | Jour 21 |
|---|---|---|---|
| Cellules témoins | 0,140 | 0,142 | 0,136 |
| Cellules témoins infectées | 1,014 | 1,510 | 0,730 |
| Cellules + VIH-1 traité 15 minutes | 0,134 | 0,150 | 0,140 |
| Cellules + VIH-1 traité 30 minutes | 0,152 | 0,170 | 0,146 |
| Cellules + VIH-1 traité 60 minutes | 0,142 | 0,162 | 0,135 |
| Cellules + VIH-1 traité 120 minutes | 0,145 | 0,168 | 0,154 |

On peut donc conclure que le 1,1'-azobisformamide protège les cultures cellulaires contre l'infectivité du VIH-1.

**c)** Examen de l'intégration du génome viral dans l'ADN chromosomique de cellules Supt-1.

On procède comme dans l'exemple 10c). La recherche des gènes "GAG", "LTR" et "ENV", a été effectuée dans des cultures cellulaires inoculées par du virus mis à incuber pendant 15 minutes avec la substance active. Cette recherche s'est avérée négative, et on peut donc en conclure que le 1,1'-azobisformamide à la dose de 35 µg/ml protège les cellules contre l'infection par le VIH-1.

**Exemple 12**

L'absence de toxicité de l'azobisformamide pour l'être humain a déjà été décrite par B.L. OSER et cons., Studies of the Safety of Azodicarbonamide as a Flour-Maturing agent, Toxicology and applied Pharmacology 7, 445-472 (1965).

Trois volontaires sains ont été, pendant 30 jours, traités par 1.500 g de la substance active, en trois prises de 500 mg par jour.

Aucun effet secondaire n'a été signalé et les paramètres hématologiques sont restés parfaitement normaux durant les trente jours du traitement expérimental.

Par ailleurs, trois patients présentant des stades différents du SIDA ont également été traités.

Le premier malade en phase terminale présentait moins de 30 lymphocytes $T_4$ par $mm^3$, des diarrhées profuses et une désorientation spatio-temporelle complète.

Le second malade en phase "ARC" présentait une population lymphocytaire $T_4$ de 190 par $mm^3$, en chute constante, ainsi que des diarrhées occasionnelles et un herpès anal.

Le troisième patient était séropositif sain, il avait une population de 350 lymphocytes $T_4$ par $mm^3$ et il ne présentait aucun signe de pathologie.

Le premier patient présentait au jour O du traitement 1850 globules blancs, dont 17 % de lymphocytes parmi lesquels 5 % présentaient le récepteur $T_4$ (soit 24 cellules). Au jour 30 du traitement les globules blancs étaient au nombre de 2.600, dont 20 % de lymphocytes parmi lesquels 9 % présentaient le récepteur $T_4$ (soit 49 cellules par $mm^3$), ce qui représente une amélioration de 100 %. Par ailleurs, la diarrhée s'est arrêtée, le patient a récupéré une possibilité de dialogue cohérent avec ses proches ainsi qu'une autonomie limitée de marche.

Le second patient avait au jour 30 280 lymphocytes $T_4$par $mm^3$, la diarrhée avait complètement disparu ainsi que l'herpès anal.

Le troisième patient présentait au jour 30 440 lymphocytes $T_4$ par $mm^3$.

Ces résultats, bien que portant sur un échantillon restreint, sont remarquables et surprenants et ils n'étaient en aucune façon à attendre de l'homme de métier moyen.

**Exemple 13**

Comprimé effervescent désinfectant, pour 100 $cm^3$ d'eau.

| | |
|---|---|
| 1,1'-Azobisdiméthylformamide | 33 mg |
| Acide citrique | 15 mg |
| Acide tartrique | 17,5 mg |
| NaH $CO_3$ | 37,5 mg |
| Avicel PH $IC_2$ | 27 mg |
| Lactose EFK | 45 mg |

Pour un comprimé : 175 mg

**Exemple 14**

Comprimé effervescent désinfectant, pour 1000 $cm^3$ d'eau.

| | |
|---|---|
| 2,2'-Azobisméthylformamidine | 330 mg |
| Acide citrique | 150 mg |
| Acide tartrique | 175 mg |
| NaH $CO_3$ | 375 mg |
| Avicel PH $IC_2$ | 180 mg |
| Lactose EFK | 522,2 mg |
| Laurylsulfate de sodium | 64 mg |
| Aerosil 200 | 3,8 mg |

Pour un comprimé : 180 mg.

**Exemple 15**

Pâte dentifrice.

A 100g d'une pâte dentifrice comprenant 4 % de ricinilate, on mélange 30 mg de 1,1'-azobisfluoroformamidine (pour 30 jours).

**Exemple 16**

Bain de bouche.

| | |
|---|---|
| Perfluorate de sodium | 8 g |
| Borax | 32 g |
| Chlorure de sodium | 20 g |
| Bicarbonate de sodium | 40 g |
| 1,1'-Azobisfluoroformamidine | 30 g |
| Essence de menthe | 3 gouttes |

1 cuillère à café dans une tasse d'eau tiède.

**Exemple 17**

Crème.

| | |
|---|---|
| 1,1'-Azobisformamide | 1 g |
| Triéthanolamine | 1 g |
| Glycérol | 2,5 g |
| Cire blanche | 2,5 g |
| Acide stéarique | 6 g |
| Huile d'amande | 7,5 g |
| Essence de lavande | 2gouttes |
| Aqua conservans ad | 50 g FMS, |

pour 50 g de crème.

**Exemple 18**

Talc.

| | |
|---|---|
| 1,1'-Azobisformamide | 2 g |

| | |
|---|---|
| Essence de lavande | 5 gouttes |
| Talc ad | 100 g, |

pour 100 g de talc.

**Exemple 19**

Savon liquide.

| | |
|---|---|
| 1-Monochloro-azobisformamidine | 3 g |
| Savon potassique | 60 g |
| Essence de lavande | 10 gouttes |
| Solution antiseptique ad | 100 g, |

pour 100 g de savon.

**Exemple 20**

Papier de toilette, bandes et tampons hygiéniques, ouate, etc...

On prépare tout d'abord une poudre à pulvériser dont la composition est par exemple la suivante :

| | |
|---|---|
| 1,1'-Azobisdiméthylformamide | 20 mg |
| Sous-gallate de bismuth | 50 g |
| Peroxyde de zinc | 100 g |
| Talc | 840 g, |

pour 1 kg de poudre à pulvériser.

On pulvérise ensuite d'une manière courante cette poudre qui adhère aux fibres des produits traités.

**Exemple 21**

Huile pour condom.

| | |
|---|---|
| Huile de silicone | 100 g |
| 1,1'-Azobisformamide | 1 g |

Les condoms sont enduits de la préparation tant sur la face interne qu'externe.

Il doit être entendu que la présente invention n'est en aucune façon limitée aux formes de réalisation décrites ci-dessus et que bien des modifications peuvent y être apportées sans sortir de son cadre.

De nombreuses autres compositions désinfectantes ou pharmaceutiques peuvent être prévues en dehors de celles données à titre d'exemple, en suivant uniquement les formulations généralement appliquées dans le domaine concerné, qu'il s'agisse de produits de nettoyage, de compositions cosmétiques, de médicaments ou autres.

## Revendications

**Revendications pour les Etats contractants suivants : AT, BE, CH, DE, DK, FR, GB, IT, LI, LU, NL, SE**

1. Dérivés azoïques répondant à la formule générale

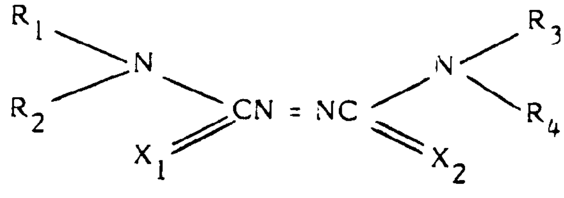

dans laquelle $R_1$ à $R_4$ sont identiques ou différents et représentent chacun un atome d'hydrogène ou d'halogène ou un radical d'hydrocarbure aliphatique ou aromatique, éventuellement substitué, comportant de 1 à 6 atomes de carbone, $X_1$ et $X_2$ sont identiques ou différents et représentent chacun un atome d'oxygène ou un groupe $NR_5$, où $R_5$ représente un atome d'hydrogène ou d'halogène, un radical d'hydrocarbure aliphatique ou aromatique comportant de 1 à 6 atomes de carbone ou un groupe nitro, $R_5$ pouvant, en présence de deux groupes $NR_5$ simultanément, présenter une signification identique ou

différente dans chacun de ces groupes et $R_5$ ayant une signification autre que celle d'atome de chlore simultanément dans les deux groupes $NR_5$ lorsque $R_1$ à $R_4$ représentent de l'hydrogène, ainsi que leurs isomères, le 1,1'-azobisdiméthylformamide étant exclu de ces dérivés, pour leur application en tant que substances thérapeutiquement actives.

2. Dérivés azoïques suivant la revendication 1, caractérisés en ce qu'ils consistent en dérivés d'azobisformamidine ou d'azobisformamide.

3. Dérivés azoïques suivant la revendication 1, choisis parmi le groupe comprenant le 1,1'-azobisformamide, la 1,1'-azobisformamidine, la 1,1'-azobisnitroformamidine, la 2,2'-azobisméthylformamidine, la 1,1'-azobisfluoroformamidine, la 1-monochloro-azobisformamidine.

4. Dérivés azoïques suivant l'une quelconque des revendications 1 à 3, pour leur mise en oeuvre dans le traitement ou la prophylaxie des maladies virales.

5. Dérivés azoïques suivant la revendication 4, pour leur mise en oeuvre dans le traitement ou la prophylaxie des infections par les virus du groupe rétrovirus, et en particulier des virus d'immunodéficience humaine VIH.

6. Composition pharmaceutique renfermant, comme substance thérapeutiquement active, au moins un dérivé azoïque suivant l'une quelconque des revendications 1 à 5, et au moins un excipient pharmaceutiquement compatible, ainsi qu'éventuellement un ou plusieurs adjuvants courants en pharmacie.

7. Composition suivant la revendication 6, caractérisée en ce qu'elle comprend, comme substance active, du 1,1'-azobisformamide, de la 1,1'-azobisformamidine, de la 1,1'-azobisnitroformamidine, la 2,2'-azobisméthylformamidine, la 1,1'-azobisfluoroformamidine, la 1-monochloro-azobisformamidine.

8. Composition suivant l'une ou l'autre des revendications 6 et 7, caractérisée en ce qu'elle comprend, en supplément, un agent désinfectant.

9. Composition suivant l'une ou l'autre des revendications 6 à 8, à administrer par voie orale ou sublinguale.

10. Composition suivant l'une ou l'autre des revendications 6 à 8, à administrer par voie rectale ou vaginale.

11. Composition suivant l'une ou l'autre des revendications 6 à 8, à administrer par injection ou perfusion.

12. Composition suivant l'une ou l'autre des revendications 6 à 8, à administrer par voie locale, transcutanée ou transmuqueuse.

13. Composition pharmaceutique suivant l'une quelconque des revendications 6 à 12, caractérisée en ce qu'elle comprend la substance active à une teneur de l'ordre de 2 mg à 350/µg/ml, en particulier de 660 µg à 330 µg/ml.

14. Procédé de préparation d'une composition pharma- ceutique suivant l'une quelconque des revendications 6 à 13, comprenant la mise en association d'au moins une substance thérapeutiquement active précitée en un excipient pharmaceutiquement compatible.

15. Utilisation de dérivés azoïques répondant à la formule générale

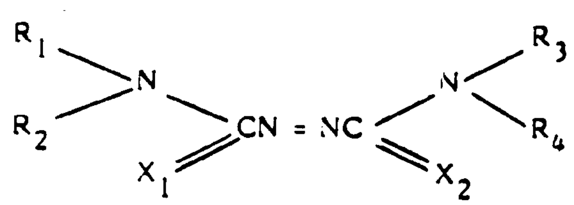

dans laquelle $R_1$ à $R_4$ sont identiques ou différents et représentent chacun un atome d'hydrogène ou d'halogène ou un radical d'hydrocarbure alphatique ou aromatique, éventuellement substitué, comportant de 1 à 6 atomes de carbone, $X_1$ et $X_2$ sont identiques ou différents et représentent chacun

un atome d'oxygène ou un groupe $NR_5$, où $R_5$ représente un atome d'hydrogène ou d'halogène, un radical d'hydrocarbure aliphatique ou aromatique comportant de 1 à 6 atomes de carbone ou un groupe nitro, $R_5$ pouvant, en présence de deux groupes $NR_5$ simultanément, présenter une signification identique ou différente dans chacun de ces groupes et $R_5$ ayant une signification autre que celle d'atome de chlore simultanément dans les deux groupes $NR_5$ lorsque $R_1$ à $R_4$ représentent de l'hydrogène, ainsi que leurs isomères, pour combattre sur et/ou dans des objets inanimés les virus, notamment du groupe rétrovirus, en particulier les virus d'immunodéficience humaine VIH.

**16.** Utilisation suivant la revendication 15, dans laquelle les dérivés sont choisis parmi le groupe comprenant du 1,1'-azobisformamide, de la 1,1'-azobisformamidine, du 1,1'-azobisdiméthylformamide, de la 1,1'-azobisnitroformamidine, de la 1,1'-azobisfluoroformamidine, de la 1-monochloroazobisformamidine.

**17.** Utilisation suivant l'une ou l'autre des revendications 15 et 16, dans laquelle on met en oeuvre une composition de désinfection d'objets inanimés, contenant au moins une substance active suivant l'une ou l'autre des revendications 15 et 16, ainsi qu'un support approprié, et éventuellement d'autres agents désinfectants ou adjuvants courants.

**18.** Utilisation d'une substance active suivant l'une ou l'autre des revendications 15 et 16 ou respectivement d'une composition suivant la revendication 17, pour la désinfection d'objets inanimés vis-à-vis des virus, notamment du groupe rétrovirus, en particulier des virus d'immunodéficience humaine VIH.

**19.** Utilisation suivant la revendication 18, dans laquelle les objets inanimés à désinfecter sont des matériels sanitaires, en matière plastique, textile ou caoutchouteuse, des instruments, appareils et vêtements médicaux et vétérinaires, des objets nécessitant une manipulation dans le domaine de l'alimentation, des instruments de cosmétologie, des véhicules sanitaires, des surfaces de sol et de paroi, des appareils sanitaires et hygiéniques, des eaux de piscine, des boissons, et des objets analogues.

**20.** Utilisation suivant la revendication 18, dans laquelle les objets inanimés à désinfecter sont des compositions cosmétiques.

**21.** Utilisation suivant la revendication 18, dans laquelle les objets inanimés à désinfecter sont des excréments, des résidus d'analyses de laboratoire, des échantillons prelevés d'un corps humain ou animal, et des objets analogues.

**22.** Utilisation suivant la revendication 18, dans laquelle les objets inanimés à désinfecter sont des produits susceptibles d'entrer en contact avec la peau et les muqueuses d'un corps humain ou animal, éventuellement porteur de virus, produits dans lesquels ou sur lesquels ladite substance active ou composition de désinfection forme simultanément un milieu de barrière à la transmis- sion des virus précités.

**23.** Utilisation suivant la revendication 22, dans laquelle les produits susceptibles d'entrer en contact avec la peau ou les muqueuses sont des gants de chirurgien ou de dentiste, des pessaires, des condoms, et des objets analogues.

**24.** Utilisation de dérivés azoïques répondant à la formule générale

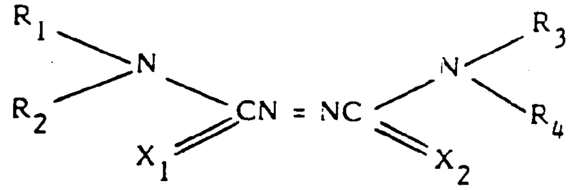

dans laquelle $R_1$ à $R_4$ sont identiques ou différents et représentent chacun un atome d'hydrogène ou d'halogène ou un radical d'hydrocarbure alphatique ou aromatique, éventuellement substitué et comportant de 1 à 6 atomes de carbone, $X_1$ et $X_2$ sont identiques ou différents et représentent chacun un atome d'oxygène ou un groupe $NR_5$, où $R_5$ représente un atome d'hydrogène ou d'halogène, un radical d'hydrocarbure aliphatique ou aromatique comportant de 1 à 6 atomes de carbone ou un groupe nitro, $R_5$ pouvant, en présence de deux groupes $NR_5$ simultanément, présenter une signification identique ou différente dans chacun de ces groupes et $R_5$ ayant une signification autre que celle d'atome de chlore

simultanément dans les deux groupes $NR_5$ lorsque $R_1$ à $R_4$ représentent de l'hydrogène, ainsi que de leurs isomères,

pour la fabrication de médicaments à mettre en oeuvre dans le traitement ou la prophylaxie des maladies virales, notamment pour les infections par les virus du groupe rétrovirus, et en particulier pour les infections par les virus d'immunodéficience humaine VIH.

**Revendications pour les Etats contractants suivants : ES, GR**

1. Procédé de préparation d'une composition pharmaceutique comprenant la mise en association, comme substance thérapeutiquement active, d'au moins un dérivé azoïque répondant à la formule générale

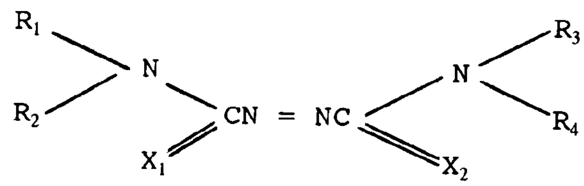

dans laquelle $R_1$ à $R_4$ sont identiques ou différents et représentent chacun un atome d'hydrogène ou d'halogène ou un radical d'hydrocarbure aliphatique ou aromatique, éventuellement substitué, comportant de 1 à 6 atomes de carbone, $X_1$ et $X_2$ sont identiques ou différents et représentent chacun un atome d'oxygène ou un groupe $NR_5$, où $R_5$ représente un atome d'hydrogène ou d'halogène, un radical d'hydrocarbure aliphatique ou aromatique comportant de 1 à 6 atomes de carbone ou un groupe nitro, $R_5$ pouvant, en présence de deux groupes $NR_5$ simultanément, présenter une signification identique ou différente dans chacun de ces groupes et $R_5$ ayant une signification autre que celle d'atome de chlore simultanément dans les deux groupes $NR_5$ lorsque $R_1$ à $R_4$ représentent de l'hydrogène, ainsi que leurs isomères, le 1,1'-azobisdiméthylformamide étant exclu de ces dérivés, et d'au moins un excipient pharmaceutiquement compatible, ainsi qu'éventuellement un ou plusieurs adjuvants courants en pharmacie.

2. Procédé suivant la revendication 1, caractérisé en ce que la composition comprend, comme substance active, du 1,1'-azobisformamide, de la 1,1'-azobisformamidine, de la 1,1'-azobisnitroformamidine , de la 2,2'-azobisméthylformamidine, de la 1,1'-azobisfluoroformamidine, de la 1-monochloro-azobisformamidine.

3. Procédé suivant l'une ou l'autre des revendications 1 et 2, caractérisé en ce qu'il comprend, en supplément, l'addition d'un agent désinfectant.

4. Procédé suivant l'une quelconque des revendications 1 à 3, caractérisé en ce qu'il comprend la mise en oeuvre de la substance active à une teneur de l'ordre de 2 mg à 350/ µg/ml, en particulier de 660 µg à 330 µg/ml.

5. Utilisation de dérivés azoïques répondant à la formule générale

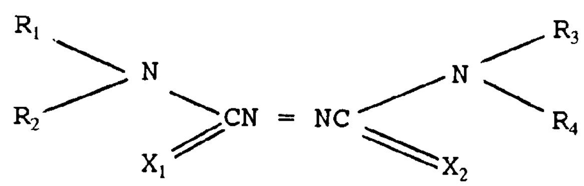

dans laquelle $R_1$ à $R_4$ sont identiques ou différents et représentent chacun un atome d'hydrogène ou d'halogène ou un radical d'hydrocarbure alphatique ou aromatique, éventuellement substitué, comportant de 1 à 6 atomes de carbone, $X_1$ et $X_2$ sont identiques ou différents et représentent chacun un atome d'oxygène ou un groupe $NR_5$, où $R_5$ représente un atome d'hydrogène ou d'halogène, un radical d'hydrocarbure aliphatique ou aromatique comportant de 1 à 6 atomes de carbone ou un groupe nitro, $R_5$ pouvant, en présence de deux groupes $NR_5$ simultanément, présenter une signification identique ou différente dans chacun de ces groupes et $R_5$ ayant une signification autre que celle d'atome de chlore simultanément dans les deux groupes $NR_5$ lorsque $R_1$ à $R_4$ représentent de l'hydrogène, ainsi que leurs

isomères, pour combattre sur et/ou dans des objets inanimés les virus, notamment du groupe rétrovirus, en particulier les virus d'immunodéficience humaine VIH.

6. Utilisation suivant la revendication 5, dans laquelle les dérivés sont choisis parmi le groupe comprenant du 1,1'-azobisformamide, de la 1,1'-azobisformamidine, du 1,1'-azobisdiméthylformamide, de la 1,1'-azobisnitroformamidine, de la 1,1'-azobisfluoroformamidine, de la 1-monochloroazobisformamidine.

7. Utilisation suivant l'une ou l'autre des revendications 5 et 6, dans laquelle on met en oeuvre une composition de désinfection d'objets inanimés, contenant au moins une substance active suivant l'une ou l'autre des revendications 15 et 16, ainsi qu'un support approprié, et éventuellement d'autres agents désinfectants ou adjuvants courants.

8. Utilisation d'une substance active suivant l'une ou l'autre des revendications 5 et 6 ou respectivement d'une composition suivant la revendication 7, pour la désinfection d'objects inanimés vis-à-vis des virus, notamment du groupe rétrovirus, en particulier des virus d'immunodéficience humaine VIH.

9. Utilisation suivant la revendication 8, dans laquelle les objets inanimés à désinfecter sont des matériels sanitaires, en matière plastique, textile ou caoutchouteuse, des instruments, appareils et vêtements médicaux et vétérinaires, des objets nécessitant une manipulation dans le domaine de l'alimentation, des instruments de cosmétologie, des véhicules sanitaires, des surfaces de sol et de paroi, des appareils sanitaires et hygiéniques, des eaux de piscine, des boissons, et des objets analogues.

10. Utilisation suivant la revendication 8, dans laquelle les objets inanimés à désinfecter sont des compositions cosmétiques.

11. Utilisation suivant la revendication 8, dans laquelle les objets inanimés à désinfecter sont des excréments, des résidus d'analyses de laboratoire, des échantillons prelevés d'un corps humain ou animal, et des objets analogues.

12. Utilisation suivant la revendication 8, dans laquelle les objets inanimés à désinfecter sont des produits susceptibles d'entrer en contact avec la peau et les muqueuses d'un corps humain ou animal, éventuellement porteur de virus, produits dans lesquels ou sur lesquels ladite substance active ou composition de désinfection forme simultanément un milieu de barrière à la transmis- sion des virus précités.

13. Utilisation suivant la revendication 12, dans laquelle les produits susceptibles d'entrer en contact avec la peau ou les muqueuses sont des gants de chirurgien ou de dentiste, des pessaires, des condoms, et des objets analogues.

14. Utilisation de dérivés azoïques répondant à la formule générale

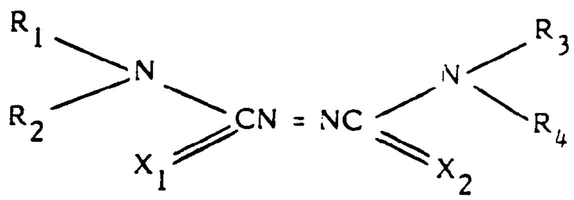

dans laquelle $R_1$ à $R_4$ sont identiques ou différents et représentent chacun un atome d'hydrogène ou d'halogène ou un radical d'hydrocarbure alphatique ou aromatique, éventuellement substitué et comportant de 1 à 6 atomes de carbone, $X_1$ et $X_2$ sont identiques ou différents et représentent chacun un atome d'oxygène ou un groupe $NR_5$, où $R_5$ représente un atome d'hydrogène ou d'halogène, un radical d'hydrocarbure aliphatique ou aromatique comportant de 1 à 6 atomes de carbone ou un groupe nitro, $R_5$ pouvant, en présence de deux groupes $NR_5$ simultanément, présenter une signification identique ou différente dans chacun de ces groupes et $R_5$ ayant une signification autre que celle d'atome de chlore simultanément dans les deux groupes $NR_5$ lorsque $R_1$ à $R_4$ représentent de l'hydrogène, ainsi que de leurs isomères,

pour la fabrication de médicaments à mettre en oeuvre dans le traitement ou la prophylaxie des maladies virales, notamment pour les infections par les virus du groupe rétrovirus, et en particulier pour les infections par les virus d'immunodéficience humaine VIH.

**Patentansprüche**

**Patentansprüche für folgende Vertragsstaaten : AT, BE, CH, DE, DK, FR, GB, IT, LI, LU, NL, SE**

1. Azoderivate der allgemeinen Formel

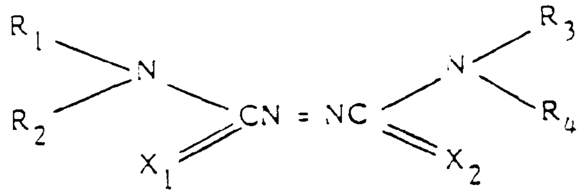

in der $R_1$ bis $R_4$ gleich oder verschieden sind und jeweils ein Wasserstoff- oder Halogenatom oder einen gegebenenfalls substituierten aliphatischen oder aromatischen Kohlenwasserstoffrest mit 1 bis 6 Kohlenstoffatomen darstellen, $X_1$ und $X_2$ gleich oder verschieden sind und jeweils ein Sauerstoffatom oder den Rest $NR_5$ darstellen, wobei $R_5$ ein Wasserstoff- oder Halogenatom, ein aliphatischer oder aromatischer Kohlenwasserstoffrest mit 1 bis 6 Kohlenstoffatomen oder eine Nitrogruppe darstellt, wobei in Gegenwart zweier $NR_5$-Reste die jeweiligen Reste $R_5$ gleiche oder verschiedene Bedeutung haben und $R_5$ nicht gleichzeitig jeweils ein Chloratom in beiden $NR_5$-Resten bedeutet, wenn $R_1$ bis $R_4$ ein Wasserstoffatom darstellen, sowie deren Isomere, mit Ausnahme von 1,1′-Azobisdimethylformamid, zur Verwendung als Arzneimittel.

2. Azoderivate nach Anspruch 1, dadurch gekennzeichnet, daß sie Azobisformamidin- oder Azobisformamid-Derivate sind.

3. Azoderivate nach Anspruch 1, gewählt aus der Gruppe bestehend aus 1,1′-Azobisformamid, 1,1′-Azobisformamidin, 1,1′-Azobisnitroformamidin, 2,2′-Azobismethylformamidin, 1,1′-Azobisfluorformamidin und 1-Monochlor-azobisformamidin.

4. Azoderivate nach einem der Ansprüche 1 bis 3 zur Verwendung bei der Behandlung von oder Vorbeugung gegen Viruserkrankungen.

5. Azoderivate nach Anspruch 4 zur Verwendung bei der Behandlung von oder Vorbeugung gegen Infektionen durch Viren vom Retrovirustyp, insbesondere durch humane Immunschwächeviren HIV.

6. Arzneimittel, umfassend als therapeutischen Wirkstoff mindestens ein Azoderivat nach einem der Ansprüche 1 bis 5 und mindestens einen pharmazeutisch verträglichen Exzipienten, sowie gegebenenfalls einen oder mehrere in der Pharmazie übliche Zusätze.

7. Zusammensetzung nach Anspruch 6, dadurch gekennzeichnet, daß sie als Wirkstoff 1,1′-Azobisformamid, 1,1′-Azobisformamidin, 1,1′-Azobisnitroformamidin, 2,2′-Azobismethylformamidin, 1,1′-Azobisfluorformamidin oder 1-Monochlor-azobisformamidin umfaßt.

8. Zusammensetzung nach einem der Ansprüche 6 oder 7, dadurch gekennzeichnet, daß sie zusätzlich ein Desinfektionsmittel umfaßt.

9. Zusammensetzung nach einem der Ansprüche 6 bis 8 zur oralen oder sublingualen Verabreichung.

10. Zusammensetzung nach einem der Ansprüche 6 bis 8 zur rektalen oder vaginalen Verabreichung.

11. Zusammensetzung nach einem der Ansprüche 6 bis 8 zur Verabreichung durch Injektion oder Perfusion.

12. Zusammensetzung nach einem der Ansprüche 6 bis 8 zur lokalen, transkutanen oder intramuskulären Verabreichung.

13. Zusammensetzung nach einem der Ansprüche 6 bis 12, dadurch gekennzeichnet, daß der Wirkstoff in einer Menge im Bereich von 2 mg bis 35 μg/ml, insbesondere von 660 μg bis 330 μg/ml, vorliegt.

14. Verfahren zur Herstellung eines Arzneimittels nach einem der Ansprüche 6 bis 13, umfassend das Vermischen mindestens eines der vorstehenden therapeutischen Wirkstoffe mit einem pharmazeutisch verträglichen Exzipienten.

15. Verwendung der Azoderivate der allgemeinen Formel

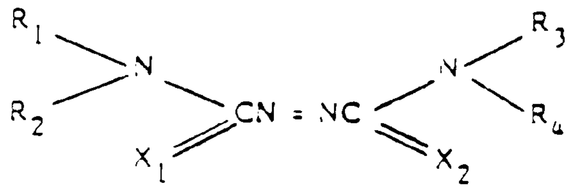

in der $R_1$ bis $R_4$ gleich oder verschieden sind und jeweils ein Wasserstoff- oder Halogenatom oder einen gegebenenfalls substituierten aliphatischen oder aromatischen Kohlenwasserstoffrest mit 1 bis 6 Kohlenstoffatomen darstellen, $X_1$ und $X_2$ gleich oder verschieden sind und jeweils ein Sauerstoffatom oder den Rest $NR_5$ darstellen, wobei $R_5$ ein Wasserstoff- oder Halogenatom, ein aliphatischer oder aromatischer Kohlenwasserstoffrest mit 1 bis 6 Kohlenstoffatomen oder eine Nitrogruppe darstellt, wobei in Gegenwart zweier $NR_5$-Reste die jeweiligen Reste $R_5$ gleiche oder verschiedene Bedeutung haben und $R_5$ nicht gleichzeitig jeweils ein Chloratom in beiden $NR_5$-Resten bedeutet, wenn $R_1$ bis $R_4$ ein Wasserstoffatom darstellen, sowie deren Isomere zur Bekämpfung von Viren, nämlich vom Retrovirustyp, insbesondere von humanen Immunschwächeviren HIV, auf und/oder in unbelebten Gegenständen.

16. Verwendung nach Anspruch 15, wobei die Derivate aus der Gruppe bestehend aus 1,1′-Azobisformamid, 1,1′-Azobisformamidin, 1,1′-Azobisdimethylformamid, 1,1′-Azobis- nitroformamidin, 1,1′-Azobisfluorformamidin und 1-Mono- chlor-azobisformamidin gewählt werden.

17. Verwendung nach einem der Ansprüche 15 oder 16, wobei man eine Zusammensetzung zur Desinfektion von unbelebten Gegenständen einsetzt, enthaltend mindestens einen Wirkstoff nach einem der Ansprüche 15 oder 16, sowie einen geeigneten Träger und gegebenenfalls weitere Desinfektionsmittel oder gängige Zusätze.

18. Verwendung eines Wirkstoffs nach einem der Ansprüche 15 oder 16 bzw. einer Zusammensetzung nach Anspruch 17 zur Desinfektion von unbelebten Gegenständen gegenüber Viren, nämlich vom Retrovirus-Typ, insbesondere humanen Immunschwächeviren HIV.

19. Verwendung nach Anspruch 18, wobei die zu desinfizierenden unbelebten Gegenstände sanitäre Materialien aus Kunststoff, Textil oder Gummi, medizinische und veterinärmedizinische Instrumente, Apparate und Kleidungsstücke, im Lebensmittelbereich zu behandelnde Gegenstände, kosmetische Instrumente, sanitäre Gegenstände, Wand- und Bodenflächen, Vorrichtungen im Gesundheits- und Hygienewesen, Schwimmbadwasser, Getränke und analoge Gegenstände sind.

20. Verwendung nach Anspruch 18, wobei die zu desinfizierenden unbelebten Gegenstände kosmetische Zusammensetzungen sind.

21. Verwendung nach Anspruch 18, wobei die zu desinfizierenden unbelebten Gegenstände Exkremente, Rückstände aus Laboranalysen, aus menschlichem oder tierischem Körper entnommene Proben oder analoge Gegenstände sind.

22. Verwendung nach Anspruch 18, wobei die zu desinfizierenden unbelebten Gegenstände Produkte sind, geeignet dazu, mit der Haut oder den Schleimhäuten eines menschlichen oder tierischen Körpers, die gegebenenfalls Virusträger sind, in Kontakt zu treten, und Produkte, in oder auf denen die wirksame Substanz oder das Desinfektionsmittel gleichzeitig eine Schutzschicht gegen das Eindringen vorstehend erwähnter Viren bilden.

23. Verwendung nach Anspruch 22, wobei die für den Kontakt mit der Haut oder den Schleimhäuten geeigneten Produkte, Handschuhe für Chirurgen und Zahnärzte, Pessare, Kondome oder analoge Gegenstände sind.

24. Verwendung von Azoderivaten der allgemeinen Formel

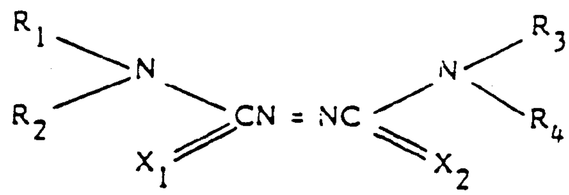

in der $R_1$ bis $R_4$ gleich oder verschieden sind und jeweils ein Wasserstoff- oder Halogenatom oder einen gegebenenfalls substituierten aliphatischen oder aromatischen Kohlenwasserstoffrest mit 1 bis 6 Kohlenstoffatomen darstellen, $X_1$ und $X_2$ gleich oder verschieden sind und jeweils ein Sauerstoffatom oder den Rest $NR_5$ darstellen, wobei $R_5$ ein Wasserstoff- oder Halogenatom, ein aliphatischer oder aromatischer Kohlenwasserstoffrest mit 1 bis 6 Kohlenstoffatomen oder eine Nitrogruppe darstellt, wobei in Gegenwart zweier $NR_5$-Reste die jeweiligen Reste $R_5$ gleiche oder verschiedene Bedeutung haben und $R_5$ nicht gleichzeitig jeweils ein Chloratom in beiden $NR_5$-Resten bedeutet, wenn $R_1$ bis $R_4$ ein Wasserstoffatom darstellen, sowie deren Isomere zur Herstellung von Arzneimitteln zur Verwendung bei der Behandlung von oder Vorbeugung gegen Viruserkrankungen, nämlich Infektionen durch Viren vom Retrovirus-Typ, insbesondere Infektionen durch den humanen Immunschwächevirus HIV.

**Patentansprüche für folgende Vertragsstaaten : ES, GR**

1. Verfahren zur Herstellung eines Arzneimittels, umfassend das Vermischen mindestens eines Azoderivats der allgemeinen Formel

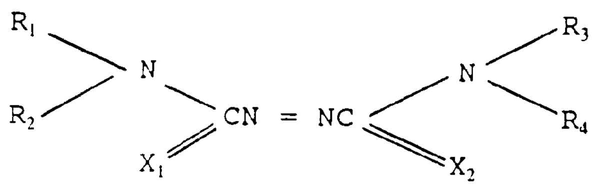

in der $R_1$ bis $R_4$ gleich oder verschieden sind und jeweils ein Wasserstoff- oder Halogenatom oder einen gegebenenfalls substituierten aliphatischen oder aromatischen Kohlenwasserstoffrest mit 1 bis 6 Kohlenstoffatomen darstellen, $X_1$ und $X_2$ gleich oder verschieden sind und jeweils ein Sauerstoffatom oder den Rest $NR_5$ darstellen, wobei $R_5$ ein Wasserstoff- oder Halogenatom, ein aliphatischer oder aromatischer Kohlenwasserstoffrest mit 1 bis 6 Kohlenstoffatomen oder eine Nitrogruppe darstellt, wobei in Gegenwart zweier $NR_5$-Reste die jeweiligen Reste $R_5$ gleiche oder verschiedene Bedeutung haben und $R_5$ nicht gleichzeitig jeweils ein Chloratom in beiden $NR_5$-Resten bedeutet, wenn $R_1$ bis $R_4$ ein Wasserstoffatom darstellen, sowie deren Isomere, mit Ausnahme von 1,1'-Azobisdimethylformamid, als Wirkstoff mit mindestens einem pharmazeutisch verträglichen Excipienten, sowie gegebenenfalls mit einem oder mehreren in der Pharmazie üblichen Zusätzen.

2. Verfahren nach Anspruch 1, dadurch gekennzeichnet, daß das Mittel als Wirkstoff 1,1'-Azobisformamid, 1,1'-Azobisformamidin, 1,1'-Azobisnitroformamidin, 2,2'-Azobismethylformamidin, 1,1'-Azobisfluorformamidin oder 1-Monochlor-azobisformamidin.

3. Verfahren nach einem der Ansprüche 1 oder 2, dadurch gekennzeichnet, daß zusätzlich ein Desinfektionsmittel vorliegt.

4. Verfahren nach einem der Ansprüche 1 bis 3, dadurch gekennzeichnet, daß der Wirkstoff in einer Menge im Bereich von 2 mg bis 35 µg/ml, insbesondere von 660 µg bis 330 µg/ml, verwendet wird.

5. Verwendung der Azoderivate der allgemeinen Formel

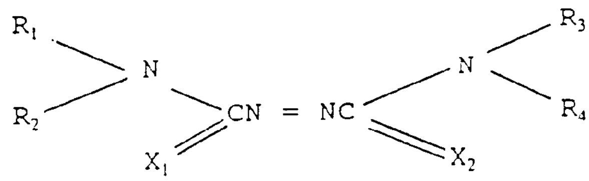

in der $R_1$ bis $R_4$ gleich oder verschieden sind und jeweils ein Wasserstoff- oder Halogenatom oder einen gegebenenfalls substituierten aliphatischen oder aromatischen Kohlenwasserstoffrest mit 1 bis 6 Kohlenstoffatomen darstellen, $X_1$ und $X_2$ gleich oder verschieden sind und jeweils ein Sauerstoffatom oder den Rest $NR_5$ darstellen, wobei $R_5$ ein Wasserstoff- oder Halogenatom, ein aliphatischer oder aromatischer Kohlenwasserstoffrest mit 1 bis 6 Kohlenstoffatomen oder eine Nitrogruppe darstellt, wobei in Gegenwart zweier $NR_5$-Reste die jeweiligen Reste $R_5$ gleiche oder verschiedene Bedeutung haben und $R_5$ nicht gleichzeitig jeweils ein Chloratom in beiden $NR_5$-Resten bedeutet, wenn $R_1$ bis $R_4$ ein Wasserstoffatom darstellen, sowie deren Isomere, zur Bekämpfung von Viren, nämlich vom Retrovirustyp, insbesondere von humanen Immunschwächeviren HIV, auf und/oder in unbelebten Gegenständen.

6. Verwendung nach Anspruch 5, wobei die Derivate aus der Gruppe bestehend aus 1,1'-Azobisformamid, 1,1'-Azobisformamidin, 1,1'-Azobisdimethylformamid, 1,1'-Azobisnitroformamidin, 1,1'-Azobisfluorformamidin und 1-Monochlor-azobisformamidin gewählt werden.

7. Verwendung nach einem der Ansprüche 5 oder 6, wobei man eine Zusammensetzung zur Desinfektion von unbelebten Gegenständen einsetzt, enthaltend mindestens einen Wirkstoff nach einem der Ansprüche 5 oder 6, sowie einen geeigneten Träger und gegebenenfalls weitere Desinfektionsmittel oder gängige Zusätze.

8. Verwendung eines Wirkstoffs nach einem der Ansprüche 5 oder 6 bzw. einer Zusammensetzung nach Anspruch 7 zur Desinfektion von unbelebten Gegenständen gegenüber Viren, nämlich vom Retrovirus-Typ, insbesondere humanen Immunschwächeviren HIV.

9. Verwendung nach Anspruch 8, wobei die zu desinfizierenden unbelebten Gegenstände sanitäre Materialien aus Kunststoff, Textil oder Gummi, medizinische und veterinärmedizinische Instrumente, Apparate und Kleidungsstücke, im Lebensmittelbereich zu behandelnde Gegenstände, kosmetische Instrumente, sanitäre Gegenstände, Wand- und Bodenflächen, Vorrichtungen im Gesundheits- und Hygienewesen, Schwimmbadwasser, Getränke und analoge Gegenstände sind.

10. Verwendung nach Anspruch 8, wobei die zu desinfizierenden unbelebten Gegenstände kosmetische Zusammensetzungen sind.

11. Verwendung nach Anspruch 8, wobei die zu desinfizierenden unbelebten Gegenstände Exkremente, Rückstände aus Laboranalysen, aus menschlichem oder tierischem Körper entnommene Proben oder analoge Gegenstände sind.

12. Verwendung nach Anspruch 8, wobei die zu desinfizierenden unbelebten Gegenstände Produkte sind, geeignet dazu, mit der Haut oder den Schleimhäuten eines menschlichen oder tierischen Körpers, die gegebenenfalls Virusträger sind, in Kontakt zu treten, und Produkte, in oder auf denen die wirksame Substanz oder das Desinfektionsmittel gleichzeitig eine Schutzschicht gegen das Eindringen vorstehend erwähnter Viren bilden.

13. Verwendung nach Anspruch 12, wobei die für den Kontakt mit der Haut oder den Schleimhäuten geeigneten Produkte, Handschuhe für Chirurgen und Zahnärzte, Pessare, Kondome oder analoge Gegenstände sind.

14. Verwendung von Azoderivaten der allgemeinen Formel

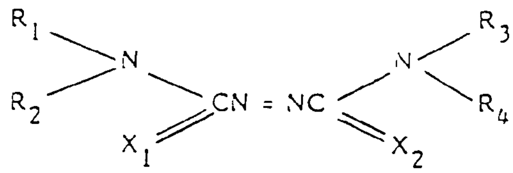

in der $R_1$ bis $R_4$ gleich oder verschieden sind und jeweils ein Wasserstoff- oder Halogenatom oder einen gegebenenfalls substituierten aliphatischen oder aromatischen Kohlenwasserstoffrest mit 1 bis 6 Kohlenstoffatomen darstellen, $X_1$ und $X_2$ gleich oder verschieden sind und jeweils ein Sauerstoffatom oder den Rest $NR_5$ darstellen, wobei $R_5$ ein Wasserstoff- oder Halogenatom, ein aliphatischer oder aromatischer Kohlenwasserstoffrest mit 1 bis 6 Kohlenstoffatomen oder eine Nitrogruppe darstellt, wobei in Gegenwart zweier $NR_5$-Reste die jeweiligen Reste $R_5$ gleiche oder verschiedene Bedeutung haben und $R_5$ nicht gleichzeitig jeweils ein Chloratom in beiden $NR_5$-Resten bedeutet, wenn $R_1$ bis $R_4$ ein Wasserstoffatom darstellen, sowie deren Isomere zur Herstellung von Arzneimitteln zur Verwendung bei der Behandlung von oder Vorbeugung gegen Viruserkrankungen, nämlich Infektionen durch Viren vom Retrovirus-Typ, insbesondere Infektionen durch den humanen Immunschwächevirus HIV.

## Claims

**Claims for the following Contracting States : AT, BE, CH, DE, DK, FR, GB, IT, LI, LU, NL, SE**

1. Azoic derivatives having the general formula :

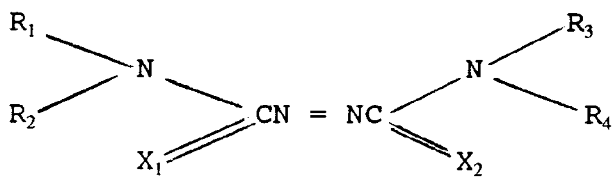

wherein $R_1$ to $R_4$ are identical or different and each represent an atom of hydrogen or halogen, or a substituted or not, aliphatic or aromatic hydrocarbon radical, comprising from 1 to 6 carbon atoms, $X_1$ and $X_2$ are identical or different and each represent an oxygen atom or a $NR_5$ group, in which $R_5$ is a hydrogen or halogen atom, an aliphatic or aromatic hydrocarbon radical comprising from 1 to 6 carbon atoms or a nitro group, $R_5$, when two $NR_5$ groups are simultaneously present, may have an identical or different meaning in each of said groups, and $R_5$ having a meaning other than an atom of chlorine simultaneously in both $NR_5$ groups when $R_1$ to $R_4$ represent hydrogen, as well as their isomers, 1.1′-azobisdimethylformamide being excluded from these derivatives, to be used as therapeutically active substances.

2. Azoic derivatives as claimed in claim 1, which are derivatives of azobisformamidine or azobisformamide.

3. Azoic derivatives as claimed in claim 1, selected in the group consisting of 1.1′-azobisformamide, 1.1′-azobisformamidine, 1.1′-azobisnitroformamidine, 2.2′-azobismethylformamidine, 1.1′-azobisfluoroformamidine, 1-monochloroazobisformamidine.

4. Azoic derivatives as claimed in any one of claims 1 to 3, to be used in the treatment or prophylaxy of viral diseases.

5. Azoic derivatives as claimed in claim 4, to be used in the treatment or prophylaxy of infections by the viruses of the retrovirus group, and in particular the human HIV immunodeficiency viruses.

6. A pharmaceutical composition comprising, as therapeutically active substance, at least one azoic derivative as claimed in any one of claims 1 to 5, and at least one pharmaceutically compatible excipient, as well as optionally one or more pharmaceutically current adjuvants.

7. A composition as claimed in claim 6, which comprises, as active substance, 1.1′-azobisformamide, 1.1′-azobisformamidine, 1.1′-azobisnitroformamidine, 2.2′-azobismethylformamidine, 1.1′-azobisfluoroformamidine, 1-monochloroazobisformamidine.

8. A composition as claimed in any one of claims 6 and 7, which comprises, as a supplement, a disinfectant agent.

9. A composition as claimed in any one of claims 6 to 8, to be administered orally or sublingually.

10. A composition as claimed in any one of claims 6 to 8, to be administered rectally or vaginally.

11. A composition as claimed in any one of claims 6 to 8, to be administered by injection or perfusion.

12. A composition as claimed in any one of claims 6 to 8, to be administered topically, transcutaneously or transmucosally.

13. A pharmaceutical composition as claimed in any one of claims 6 to 12, which comprises the active substance in an amount of about 2 mgr to 35 μgr/ml, in particular 660 μgr to 330 μgr/ml.

14. A process of preparation of a pharmaceutical composition as claimed in any one of claims 6 to 13, comprising association of at least one therapeutically active substance as mentioned before and of a pharmaceutically compatible excipient.

15. Use of azoic derivatives of the general formula :

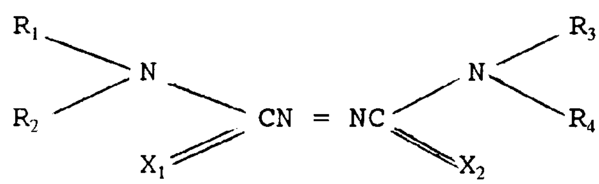

wherein $R_1$ to $R_4$ are identical or different and each represent an atom of hydrogen or halogen, or a substituted or not, aliphatic or aromatic hydrocarbon radical, comprising from 1 to 6 carbon atoms, $X_1$ and $X_2$ are identical or different and each represent an oxygen atom or a $NR_5$ group, in which $R_5$ is a hydrogen or halogen atom, an aliphatic or aromatic hydrocarbon radical comprising from 1 to 6 carbon atoms or a nitro group, $R_5$, when two $NR_5$ groups are simultaneously present, may have an identical or different meaning in each of said groups, and $R_5$ having a meaning other than an atom of chlorine simultaneously in both $NR_5$ groups when $R_1$ to $R_4$ represent hydrogen, as well as their isomers, for combatting on and/or in inanimate objects with the viruses, in particular of the retrovirus group, more particularly the human HIV immunodeficiency viruses.

16. Use as claimed in claim 15, wherein the derivatives are selected in the group consisting of 1.1′-azobisformamide, 1.1′-azobisformamidine, 1.1'-azobisdimethylformamide, 1.1′-azobisnitroformamidine, 1.1′-azobisfluoroformamidine, 1-monochloroazobisformamidine.

17. Use as claimed in any one of claims 15 and 16, comprising an application of a composition for disinfecting inanimate objects, comprising at least one active substance as claimed in any one of claims 15 and 16, as well as a suitable vehicle, and optionally other current disinfectant or adjuvant agents.

18. Use of an active substance as claimed in any one of claims 15 and 16, or of a composition as claimed in claim 17 respectively, for disinfecting inanimate objects against viruses, in particular of the retrovirus group, more particularly human HIV immunodeficiency viruses.

19. Use as claimed in claim 18, in which the inanimate objects to be disinfected are plastic, textile or rubber sanitary materials, medical and veterinary instruments, apparatuses and clothes, objects requiring a handling in the alimentary field, cosmetology instruments, sanitary vehicles, ground and wall surfaces, sanitary and hygienic apparatuses, water for swimming pools, beverages and analogous objects.

20. Use as claimed in claim 18, in which the inanimate objects to be disinfected are cosmetic compositions.

21. Use as claimed in claim 18, in which the inanimate objects to be disinfected are excrements, wastes of

analysis laboratories, samples taken off from a human or animal body, and similar articles.

**22.** Use as claimed in claim 18, in which the inanimate objects to be disinfected are products which can come into contact with the skin and the mucosas of a human or animal body, which is optionally a virus carrier, products in or on which said active substance or composition of disinfection simultaneously forms a barrier medium against the transmission of said viruses.

**23.** Use as claimed in claim 22, wherein the products able to come into contact with the skin or the mucosas are surgeon or dentist gloves, pessaries, preservative sheaths and similar articles.

**24.** Use of azoic derivatives of the general formula :

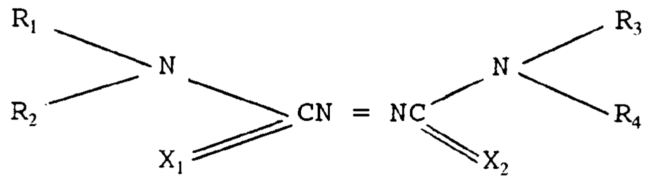

wherein $R_1$ to $R_4$ are identical or different and each represent an atom of hydrogen or halogen, or a substituted or not, aliphatic or aromatic hydrocarbon radical, comprising from 1 to 6 carbon atoms, $X_1$ and $X_2$ are identical or different and each represent an oxygen atom or a $NR_5$ group, in which $R_5$ is a hydrogen or halogen atom, an aliphatic or aromatic hydrocarbon radical comprising from 1 to 6 carbon atoms or a nitro group, $R_5$, when two $NR_5$ groups are simultaneously present, may have an identical or different meaning in each of said groups, and $R_5$ having a meaning other than an atom of chlorine simultaneously in both $NR_5$ groups when $R_1$ to $R_4$ represent hydrogen, as well as their isomers, for manufacturing drugs to be used in the treatment or prophylaxy of viral diseases, in particular against the infections by the viruses of the retrovirus group, more particularly for the infections by human HIV immunodeficiency viruses.

**Claims for the following Contracting States : ES, GR**

**1.** A process of preparation of a pharmaceutical composition comprising association of, as therapeutically active substance, at least one azoic derivative having the general formula :

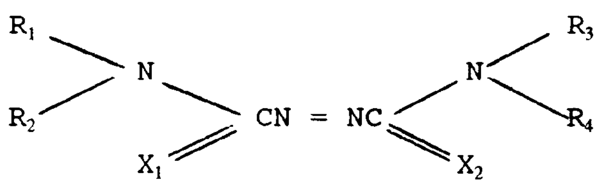

wherein $R_1$ to $R_4$ are identical or different and each represent an atom of hydrogen or halogen, or a substituted or not, aliphatic or aromatic hydrocarbon radical, comprising from 1 to 6 carbon atoms, $X_1$ and $X_2$ are identical or different and each represent an oxygen atom or a $NR_5$ group, in which $R_5$ is a hydrogen or halogen atom, an aliphatic or aromatic hydrocarbon radical comprising from 1 to 6 carbon atoms or a nitro group, $R_5$, when two $NR_5$ groups are simultaneously present, may have an identical or different meaning in each of said groups, and $R_5$ having a meaning other than an atom of chlorine simultaneously in both $NR_5$ groups when $R_1$ to $R_4$ represent hydrogen, as well as their isomers, 1.1′-azobisdimethylformamide being excluded from these derivatives, and at least one pharmaceutically compatible excipient, as well as optionally one or more pharmaceutically current adjuvants.

**2.** A process as claimed in claim 1, wherein the composition comprises, as active substance, 1.1'-azobisformamide, 1.1′-azobisformamidine, 1.1′-azobisnitroformamidine, 2.2′-azobismethylformamidine, 1.1′-azobisfluoroformamidine, 1-monochloroazobisformamidine.

**3.** A process as claimed in any one of claims 1 and 2, which comprises, as a supplement, an addition of a disinfectant agent.

4. A process as claimed in any one of claims 1 to 3, which comprises the application of the active substance in an amount of about 2 mg to 35 µgr/ml, in particular 660 µgr to 330 µgr/ml.

5. Use of azoic derivatives of the general formula :

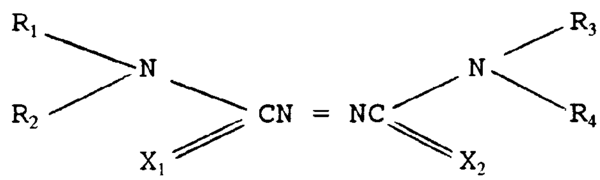

wherein $R_1$ to $R_4$ are identical or different and each represent an atom of hydrogen or halogen, or a substituted or not, aliphatic or aromatic hydrocarbon radical, comprising from 1 to 6 carbon atoms, $X_1$ and $X_2$ are identical or different and each represent an oxygen atom or a $NR_5$ group, in which $R_5$ is a hydrogen or halogen atom, an aliphatic or aromatic hydrocarbon radical comprising from 1 to 6 carbon atoms or a nitro group, $R_5$, when two $NR_5$ groups are simultaneously present, may have an identical or different meaning in each of said groups, and $R_5$ having a meaning other than an atom of chlorine simultaneously in both $NR_5$ groups when $R_1$ to $R_4$ represent hydrogen, as well as their isomers, for combatting on and/or in inanimate objects with the viruses, in particular of the retrovirus group, more particularly the human HIV immunodeficiency viruses.

6. Use as claimed in claim 5, wherein the derivatives are selected in the group consisting of 1.1'-azobisformamide, 1.1'-azobisformamidine, 1.1'-azobisdimethylformamide, 1.1'-azobisnitroformamidine, 1.1'-azobisfluoroformamidine, 1-monochloroazobisformamidine.

7. Use according to any one of claims 5 and 6, comprising an application of a composition for disinfecting inanimate objects, comprising at least one active substance as claimed in any one of claims 5 and 6, as well as a suitable vehicle, and optionally other current disinfectant or adjuvant agents.

8. Use of an active substance as claimed in any one of claims 5 and 6, or of a composition as claimed in claim 7 respectively, for disinfecting inanimate objects against viruses, in particular of the retrovirus group, more particularly human HIV immunodeficiency viruses.

9. Use as claimed in claim 8, in which the inanimate objects to be disinfected are plastic, textile or rubber sanitary materials, medical and veterinary instruments, apparatuses and clothes, objects requiring a handling in the alimentary field, cosmetology instruments, sanitary vehicles, ground and wall surfaces, sanitary and hygienic apparatuses, water for swimming pools, beverages and analogous objects.

10. Use as claimed in claim 8, in which the inanimate objects to be disinfected are cosmetic compositions.

11. Use as claimed in claim 8, in which the inanimate objects to be disinfected are excrements, wastes of analysis laboratories, samples taken off from a human or animal body, and similar articles.

12. Use as claimed in claim 8, in which the inanimate objects to be disinfected are products which can come into contact with the skin and the mucosas of a human or animal body, which is optionally a virus carrier, products in or on which said active substance or composition of disinfection simultaneously forms a barrier medium against the transmission of said viruses.

13. Use as claimed in claim 12, wherein the products able to come into contact with the skin or the mucosas are surgeon or dentist gloves, pessaries, preservative sheaths and similar articles.

14. Use of azoic derivatives of the general formula :

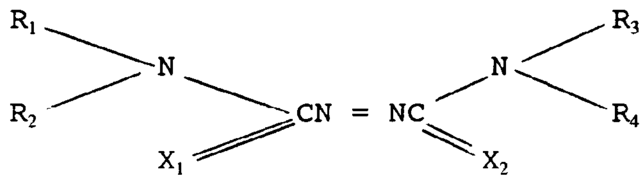

wherein $R_1$ to $R_4$ are identical or different and each represent an atom of hydrogen or halogen, or a substituted or not, aliphatic or aromatic hydrocarbon radical, comprising from 1 to 6 carbon atoms, $X_1$ and $X_2$ are identical or different and each represent an oxygen atom or a $NR_5$ group, in which $R_5$ is a hydrogen or halogen atom, an aliphatic or aromatic hydrocarbon radical comprising from 1 to 6 carbon atoms or a nitro group, $R_5$, when two $NR_5$ groups are simultaneously present, may have an identical or different meaning in each of said groups, and $R_5$ having a meaning other than an atom of chlorine simultaneously in both $NR_5$ groups when $R_1$ to $R_4$ represent hydrogen, as well as their isomers, for manufacturing drugs to be used in the treatment or prophylaxy of viral diseases, in particular against the infections by the viruses of the retrovirus group, more particularly for the infections by human HIV immunodeficiency viruses.